# EUROPEAN PATENT APPLICATION

(11) **EP 3 483 165 A1**
(43) Date of publication of application: **15.05.2019**
(21) Application number: 17823604.8
(22) Date of filing: 04.07.2017
(51) Int. Cl.: C07D 493/22, A01N 43/90, A01P 7/02, A01P 7/04

(54) **CRYSTAL FORM A OF MILBEMYCIN A3 OXIME AND PREPARATION METHOD THEREOF**

(30) Priority: 06.07.2016 CN 201610541333
(71) Applicant: Zhejiang Hisun Pharmaceutical Co., Ltd., Taizhou, Zhejiang 318000 (CN)
(72) Inventor: TENG, Yun, Taizhou Zhejiang 318000 (CN); XU, Hongbing, Taizhou Zhejiang 318000 (CN); JIANG, Lianqing, Taizhou Zhejiang 318000 (CN); CHEN, Zhengjie, Taizhou Zhejiang 318000 (CN); BAI, Hua, Taizhou Zhejiang 318000 (CN)
(74) Representative: Dehns
(86) International application number: PCT/CN2017/091624
(87) International publication number: WO 2018/006792

(57) **Abstract**

The present invention relates to the field of pharmaceuticals, especially to the field of veterinary drugs, and more particularly to a crystal form of milbemycin A3 oxime, able to be characterized by the X-ray powder diffraction spectrogram, infrared absorption (IR) spectrogram, differential scanning calorimetry (DSC), and thermogravimetric analysis (TGA), etc. Furthermore, the present invention also relates to a method for preparing the crystal form of milbemycin A3 oxime and to a use of the crystal form of milbemycin A3 oxime as an insecticidal and acaricidal agent.

## Description

### Technical field

The invention relates to the field of pharmacy, especially to the field of veterinary medicine. More specifically, the present invention relates to crystal form A of Milbemycin A3 oxime and a process for preparation of the crystal form and the use of the crystal form as an insecticide or an acaricide.

### Technical Background

Milbemycins is a pesticide of microbial natural product, which has been shown to be one of the most excellent acaricides in the world today. The US Environmental Protection Agency identified it as a low-risk pesticide, and the Netherlands approved it as "GNO" (a natural product in crop production). It is an eco-friendly pesticide and is suitable for the comprehensive prevention and control of organic agricultural pests and diseases. It has become a popular insecticide or acaricide in developed countries.

Milbemycins is a metabolite with an insect resistance activity selected from the fermentation broth of microorganisms by the Japanese Sankyo Company with the Tetrangchus urticae Koch as a test insect (see US 3,950,360). After extensive basic research, a mixture of Milbemycins A3 and A4 components was used as an acaricide in 1983. Milbemycin oxime A3/A4 as the semi-synthetic compound of Milbemycins have also been commercialized for veterinary medicine.

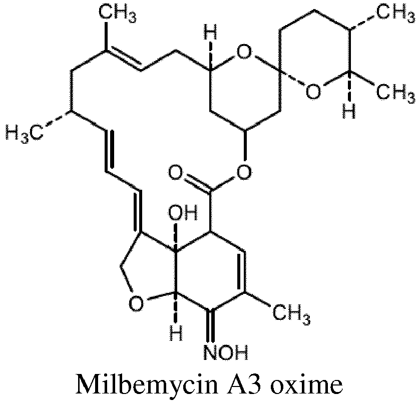

The prior art reports on Milbemycin oxime have mainly focused on the fermentation and strain breeding of Milbemycins, while the study on Milbemycin oxime has mainly focused on a mixture of Milbemycin A4 oxime and Milbemycin A3 oxime with the ratio of approximately 4:1. There is currently no report on crystal form A of Milbemycin A3 oxime as a single component. At the same time, the prior art does not mention whether the A3/A4 oxime mixture belongs to a substance having a crystal form, and the most recent patent CN201410606012.9 does not mention to which crystal form the mixture belongs. As appreciated by those skilled in the art, a crystal form of a single component has many advantages in pharmaceutics and pharmacodynamics, such as stable nature, ease of storage, ease of dissolution, ease of dispersion, ability to form a homogeneous solution, and the like. Since it is difficult for the mixture of A3/A4 oxime to form a crystal form, a single component of A3 oxime is prepared and a crystal form is obtained in the present invention.

### Summary

One object of the present invention to provide a new crystal form of Milbemycin A3 oxime, and the crystal form is designated as crystal form A.

The crystal form A of Milbemycin A3 oxime of the present invention, using Cu-Kα radiation, has characteristic peaks at the following 2θ angles in the X-ray powder diffraction pattern: 5.32±0.20°, 8.39±0.20°, 13.98±0.20°, 14.45 ±0.20°, 15.36±0.20°, 16.50±0.20°, 18.61±0.20°, 25.95±0.20°, and 26.28±0.20°.

Further, the crystal form A of Milbemycin A3 oxime of the present invention also has characteristic peaks at the following 2θ diffraction angles: 6.99±0.20°, 10.67±0.20°, 12.99±0.20°, 13.22±0.20°, 14.76±0.20°, 17.97± 0.20°, 19.09±0.20°, 20.01±0.20°, 20.65±0.20°, 22.36±0.20°, 22.79±0.20°, 24.10±0.20°, 27.42±0.20°, and 28.35±0.20°.

The 2θ angle, d value and relative intensity data of the crystal form A of Milbemycin A3 oxime of the present invention are shown in Table 1:

**Table 1**

| Peak No. | 2θ (°) | d (Å) | Relative intensity (%) |
|---|---|---|---|
| 1 | 5.32 | 16.60 | 50.6 |
| 2 | 6.99 | 12.64 | 16.6 |
| 3 | 8.39 | 10.53 | 40.3 |
| 4 | 10.67 | 8.28 | 10.9 |
| 5 | 12.99 | 6.81 | 14.4 |
| 6 | 13.22 | 6.69 | 22.5 |
| 7 | 13.98 | 6.29 | 42.5 |
| 8 | 14.45 | 6.12 | 12.8 |
| 9 | 14.76 | 6.00 | 8.4 |
| 10 | 15.36 | 5.76 | 79.4 |
| 11 | 16.50 | 5.37 | 100 |
| 12 | 17.97 | 4.93 | 19.4 |
| 13 | 18.61 | 4.76 | 43.4 |
| 14 | 19.09 | 4.65 | 28.8 |
| 15 | 20.01 | 4.43 | 24.4 |
| 16 | 20.65 | 4.30 | 5.9 |
| 17 | 22.36 | 3.97 | 13.4 |
| 18 | 22.79 | 3.90 | 14.1 |
| 19 | 24.10 | 3.70 | 5.9 |
| 20 | 25.95 | 3.43 | 20.6 |
| 21 | 26.28 | 3.39 | 14.1 |
| 22 | 27.42 | 3.25 | 7.5 |
| 23 | 28.35 | 3.15 | 6.9 |

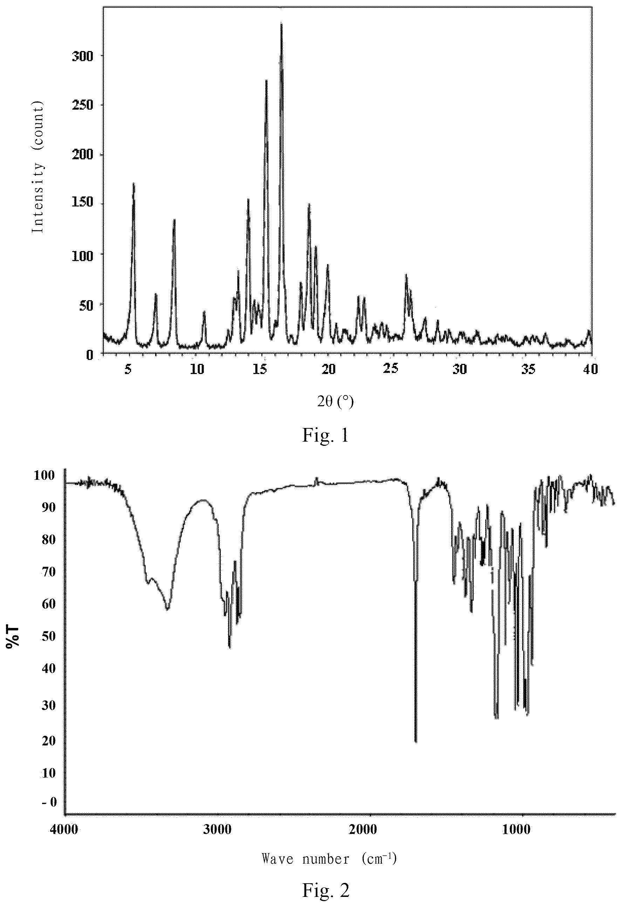

The XRPD pattern of the crystal form A of Milbemycin A3 oxime of the present invention is shown in FIG 1.

The crystal form A of Milbemycin A3 oxime of the present invention can also be tableted by using KBr so as to obtain an infrared absorption spectrum. According to the spectrum, it has characteristic peaks at the following positions: 3334 cm⁻¹, 2950 cm⁻¹, 2876 cm⁻¹, 2858 cm⁻¹, 1703 cm⁻¹, 1450 cm⁻¹, 1429 cm⁻¹, 1376 cm⁻¹, 1336 cm⁻¹, 1316 cm⁻¹, 1269 cm⁻¹, 1255 cm⁻¹, 1222 cm⁻¹, 1181 cm⁻¹, 1168 cm⁻¹, 1116 cm⁻¹, 1089 cm⁻¹, 1054 cm⁻¹, 1033 cm⁻¹, 988 cm⁻¹, 973 cm⁻¹ and 942 cm⁻¹.

Further, the crystal form A of Milbemycin A3 oxime of the present invention has further characteristic peaks at 900 cm⁻¹, 866 cm⁻¹, 850 cm⁻¹, 821 cm⁻¹, 791 cm⁻¹, 772 cm⁻¹, 717 cm⁻¹, 583 cm⁻¹, 533 cm⁻¹, 488 cm⁻¹ and 461 cm⁻¹.

The infrared spectrum of the crystal form A of Milbemycin A3 oxime of the present invention is shown in Fig. 2.

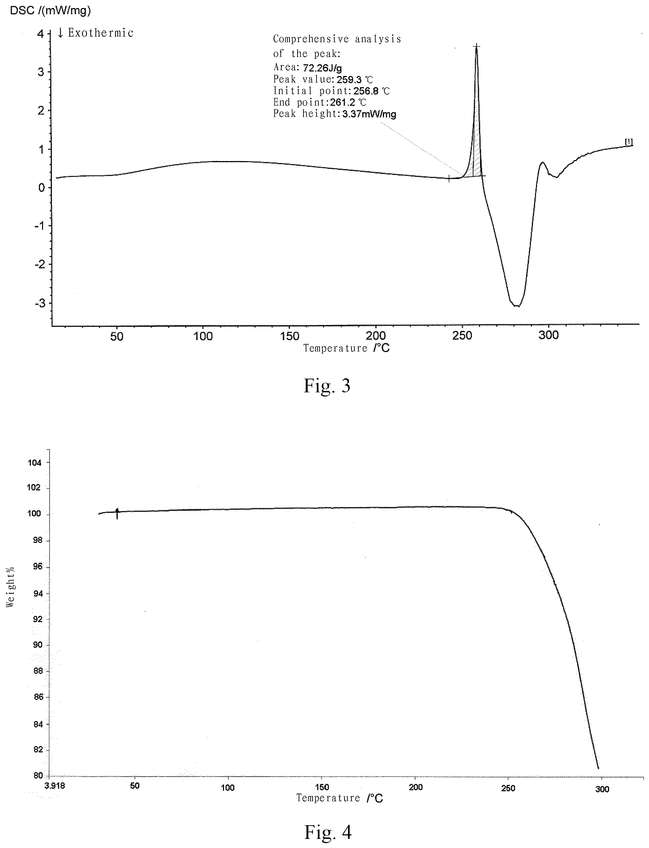

The differential scanning calorimetry pattern of the crystal form A of Milbemycin A3 oxime of the present invention is shown in FIG 3.

The differential scanning calorimetry pattern of the crystal form A of Milbemycin A3 oxime of the present invention exhibits an exothermic peak at 256.8-261.2 °C.

The thermogravimetric analysis pattern of the crystal form A of Milbemycin A3 oxime of the present invention is shown in FIG 4.

Another object of the present invention is to provide a process for the preparation of the crystal form A of Milbemycin A3 oxime, which comprises steps of:
(1) dissolving Milbemycin A3 oxime in a solvent to form a solution;
(2) adding the solution obtained in step (1) to an anti-solvent;
(3) crystallizing, filtrating and vacuum drying to obtain the crystal form A of Milbemycin A3 oxime.

Further, the solvent of the present invention is selected from methanol, ethanol, acetone, acetonitrile, DMF, n-propanol, isopropanol, n-butanol, isobutanol, or chloroform, and the anti-solvent is water or n-heptane.

Further, the concentration of Milbemycin A3 oxime in a solvent is 100 g/L to 500 g/L, preferably 200 g/L to 400 g/L.

Further, the temperature of the solution obtained in step (1) is controlled to be 50 °C to 80 °C, preferably 60 °C to 70 °C.

Further, after the solution obtained in step (1) is added to the anti-solvent, the volume content of the anti-solvent in the mixed liquid is 70% to 90%, preferably 75% to 85%.

Further, the temperature of the mixed liquid obtained in step (2) is controlled to be 30 °C to 60 °C, preferably 40 °C to 50 °C.

Further, the vacuum drying temperature in step (3) is controlled to be 50 °C to 80 °C, preferably 60 °C to 70 °C, and the drying time is controlled to be 24-60 h, preferably 36-48 h.

In a preferred embodiment, prior to step (3), a step of adding seed crystals of the crystal form A of Milbemycin A3 oxime to the mixed liquid obtained in step (2) is further included.

Still another object of the present invention is to provide use of the crystal form A of Milbemycin A3 oxime in the preparation of an insecticide or an acaricide.

It should be emphasized that, the meaning of or the scope intended to be protected by the numerical values or numerical endpoints referred to in the technical solutions of the present invention are not limited to the numbers themselves. Those skilled in the art will appreciate that they encompass those allowable ranges of error that have been widely accepted in the art, such as experimental errors, measurement errors, statistical errors, and random errors, etc., which are included within the scope of the present invention.

### Brief description of the drawings

Figure 1 is an XRPD pattern of the crystal form A of Milbemycin A3 oxime obtained in Example 1 of the present invention.
Figure 2 is an infrared spectrum of the crystal form A of Milbemycin A3 oxime obtained in Example 1 of the present invention.
Figure 3 is a differential scanning calorimetry thermogram of the crystal form A of Milbemycin A3 oxime obtained in Example 1 of the present invention.
Figure 4 is a thermogravimetric analysis thermogram of the crystal form A of Milbemycin A3 oxime obtained in Example 1 of the present invention.
Figure 5 is an XRPD pattern of a mixture of Milbemycin A3/A4 oxime.
Figure 6 is a differential scanning calorimetry thermogram of a mixture of Milbemycin A3/A4 oxime.

### Embodiments

The following examples further describe the invention in detail, but they are not intended to limit or restrict the scope of the invention. The X-ray powder diffraction instrument and test conditions of the present invention are as follows:
X-diffraction instrument model Rigaku D/max-2200 Cu target;
method of operation: scanning speed 4°/min, scanning step width 0.01°, operating temperature 25 ± 1 °C, wavelength 1.54056 Å.

The infrared spectrophotometer and the test conditions of the invention are as follows:
infrared spectrophotometer model: BRWKER VECTOR 22;
method of operation: KBr tableting method is used, and the scanning range is 400-4000 cm⁻¹.

The differential scanning calorimetry (DSC) test conditions of the present invention are:
DSC detector model is: PERKIN ELMER DSC8000;
method of operation: heating rate 10 °C/min, temperature range: 20 °C - 280 °C.

The thermogravimetric analysis (TGA) test conditions of the present invention are:
TGA model: PerkinElmer TGA400;
method of operation: heating rate 10 °C/min, temperature range: 30 °C - 300 °C.

### Examples

### Preparation of Milbemycin A3 oxime

Streptomyces hygroscopicus (CGMCC NO. 9672) was inoculated into a fermentation medium, fermentation medium formula (g/L): yeast extract 5, sucrose 120, skim milk powder 10, soy cake powder 10, cottonseed cake powder 14, dipotassium hydrogen phosphate 1, ferrous sulfate heptahydrate 0.1, zinc sulfate 0.02, calcium carbonate 5, copper sulfate 0.05, sodium molybdate 0.5. 0.25% antifoamer was added as a defoaming agent into a 50L fermenter with a feeding volume of 35L. The pH was 7.2-7.6 before sterilization, and steam sterilization was conducted at 121 °C for 25 minutes. After cooling, about 3.5L culture solution from seed tank was inoculated. Fermentation was carried out at a temperature of 28 °C ± 1 °C, and the lower limit of stirring paddle speed was 150 rpm. The stirring speed was associated with dissolved oxygen, and the dissolved oxygen was not less than 35%. The aeration rate was 0.6 vvm, fermentation cultivation for 14 days, and the fermenter was finished to obtain 30 L of fermentation broth, wherein the content of Milbemycin A3 was about 3000 mg/L.

The mycelium was obtained by filtration of the fermentation broth, and the mycelium was soaked with acetone, and the soaking liquid was distilled under a reduced pressure until being completely volatilized to obtain 220 g of a brown substance. This substance was extracted with hot n-hexane, and the extract was distilled under a reduced pressure until being completely volatilized. The obtained solid was dissolved in methanol, and the solution was allowed to stand for precipitation at -20 °C for 24 hours and the precipitate was removed. The supernatant was distilled under a reduced pressure until the solvent was completely volatilized to yield 180 g of oil. The oil was separated by an alumina chromatographic column, being eluted with chloroform to give 50 g of crude product containing Milbemycin A3/A4.

20 g of the above crude product containing Milbemycin A3/A4 was dissolved in 400 ml of dichloromethane, the obtained solution was added dropwise on an ice bath into dichloromethane containing 40 g of potassium chromate and 64 g of pyridine, and stirred for 1 h. The obtained mixed solution was then mixed with 5.6 L of n-hexane and filtered. The filtrate was distilled under a reduced pressure, and then was separated by a silica gel column, being eluted with 90:10 of n-hexane/ethyl acetate to afford 12 g of a mixture of MilbemycinA3/A4 ketone.

3.75 g of hydroxylamine hydrochloride was added to 350 mL of cyclohexane containing 3 g of acetic acid and 5 g of sodium acetate under 10-15 °C, being stirred for 5 minutes. 10 g of the above mixture of Milbemycin A3/A4 ketone was added to the mixed liquid and stirred at room temperature for 1 h. Then 5 g of sodium acetate and 3.75 g of hydroxylamine hydrochloride were further added. Stirring was continued for 40 minutes. 10 mL of water was added dropwise to the mixture and stirred for 2.5 hours. The mixture was distilled under a reduced pressure to 1/2 of the original volume, then was diluted with water and extracted with ethyl acetate. The extract was distilled under a reduced pressure and separated by a silica gel column, being eluted with 3:2 of n-hexane/ethyl acetate to give 4 g of a mixture of Milbemycin A3/A4 oxime.

10 mL of methanol solution in which 3.0 g of a mixture of Milbemycin A3/A4 oxime was dissolved was added to a preparative liquid chromatograph (purchased from Innovation Tongheng), and ODS bonded silica reverse phase packing was used. The eluent was methanol : water = 90 : 10, and Milbemycin A3 oxime fraction was collected and concentrated at 60 °C, cooled, then precipitated by adding water, filtered to obtain a wet product of Milbemycin A3 oxime.

### Preparation of the crystal form A of Milbemycin A3 oxime

### Example 1:

2.0 g of the wet product of Milbemycin A3 oxime was added to 5 ml of ethanol, stirred and heated to 50 °C, and the wet product was completely dissolved. The solution was added to 15 ml of water. After the addition, the temperature of the mixed liquid was controlled to be 40 °C, and 1.0 g of wet crystals were obtained by filtration, and the crystals were dried under vacuum at 70 °C for 48 h.

Through measuring by X-ray powder diffraction, infrared, DSC and TGA, the crystals were determined to be crystal form A of Milbemycin A3 oxime, and the X-ray powder diffraction pattern, infrared spectrum, DSC thermogram and TGA thermogram are shown in Figures 1 to 4, respectively.

### Example 2:

2.0 g of the wet product of Milbemycin A3 oxime was added to 5 ml of ethanol, stirred and heated to reflux, and the wet product was completely dissolved. The solution was added to 15 ml of water. After the addition, the temperature of the mixed liquid was controlled to be 50 °C, and 0.8 g of wet crystals were obtained by filtration, and the crystals were dried under vacuum at 70 °C for 48 h. Upon measuring, crystal form A of Milbemycin A3 oxime was obtained.

### Example 3:

1.5 g of the wet product of Milbemycin A3 oxime was added to 5 ml of ethanol, stirred and heated to 60 °C, and the wet product was completely dissolved. The solution was added to 15 ml of water. After the addition, the temperature of the mixed liquid was controlled to be 45 °C, and 0.8 g of wet crystals were obtained by filtration, and the crystals were dried under vacuum at 70 °C for 48 h. Upon measuring, crystal form A of Milbemycin A3 oxime was obtained.

### Example 4:

2.0 g of the wet product of Milbemycin A3 oxime was added to 5 ml of anhydrous methanol, stirred and heated to reflux, and the wet product was completely dissolved. The solution was added to 20 ml of water. After the addition, the temperature of the mixed liquid was controlled to be 50 °C, and 0.9 g of wet crystals were obtained by filtration, and the crystals were dried under vacuum at 60 °C for 48 h. Upon measuring, crystal form A of Milbemycin A3 oxime was obtained.

### Example 5:

2.0 g of the wet product of Milbemycin A3 oxime was added to 6 ml of acetonitrile, heated to 70 °C with stirring, and the wet product was completely dissolved. The solution was added to 18 ml of water. After the addition, the temperature of the mixed liquid was controlled to 40 °C, and 0.7 g of wet crystals were obtained by filtration, and the crystals were dried under vacuum at 60 °C for 48 h. Upon measuring, crystal form A of Milbemycin A3 oxime was obtained.

### Example 6

2.0 g of the wet product of Milbemycin A3 oxime was added to 5 ml of acetone, and the mixture was heated to 60 °C with stirring, and the wet product was completely dissolved. The solution was added to 18 ml of water. After the addition, the temperature of the mixed liquid was controlled to be 35 °C, and 0.9 g of wet crystals were obtained by filtration, and the crystals were dried under vacuum at 80 °C for 36 h. Upon measuring, crystal form A of Milbemycin A3 oxime was obtained.

### Example 7

1.0 g of the wet product of Milbemycin A3 oxime was added to 5 ml of DMF, heated to 70 °C with stirring, and the wet product was completely dissolved. The solution was added to 25 ml of water. After the addition, the temperature of the mixed liquid was controlled to be 45 °C, and 0.5 g of wet crystals were obtained by filtration, and the crystals were dried under vacuum at 70 °C for 48 h. Upon measuring, crystal form A of Milbemycin A3 oxime was obtained.

### Example 8

1.0 g of the wet product of Milbemycin A3 oxime was added to 6 ml of ethanol, stirred and heated to reflux, and the wet product was completely dissolved. The solution was added to 50 ml of n-heptane, and the seed crystals of the crystal form A of Milbemycin A3 oxime prepared in Example 2 were added. After the addition, the temperature of the mixed liquid was controlled to be 45 °C, and 0.7 g of wet crystals were obtained by filtration, and the crystals were dried at 55 °C under vacuum for 55h. Upon measuring, crystal form A of Milbemycin A3 oxime was obtained.

### Example 9

2.0 g of the wet product of Milbemycin A3 oxime was added to 5 ml of ethanol, stirred and heated to reflux, and the wet product was completely dissolved. The solution was added to 45 ml of n-heptane, and the seed crystals of the crystal form A of Milbemycin A3 oxime prepared in Example 2 was added. After the addition, the temperature of the mixed liquid was controlled to be 45 °C, and 1.5 g of wet crystals were obtained by filtration, and the crystals were dried at 70 °C under vacuum for 48h. Upon measuring, crystal form A of Milbemycin A3 oxime was obtained.

### Example 10:

1.5 g of the wet product of Milbemycin A3 oxime was added to 5 ml of isopropanol, heated to reflux with stirring, and the wet product was completely dissolved. The solution was added to 45 ml of n-heptane, and the seed crystals of the crystal form A of Milbemycin A3 oxime prepared in Example 2 was added. After the addition, the temperature of the mixed liquid was controlled to be 45 °C, and 1 g of the wet crystals were obtained by filtration, and the crystals were dried at 80 °C under vacuum for 40 h. Upon measuring, crystal form A of Milbemycin A3 oxime was obtained.

### Stability test

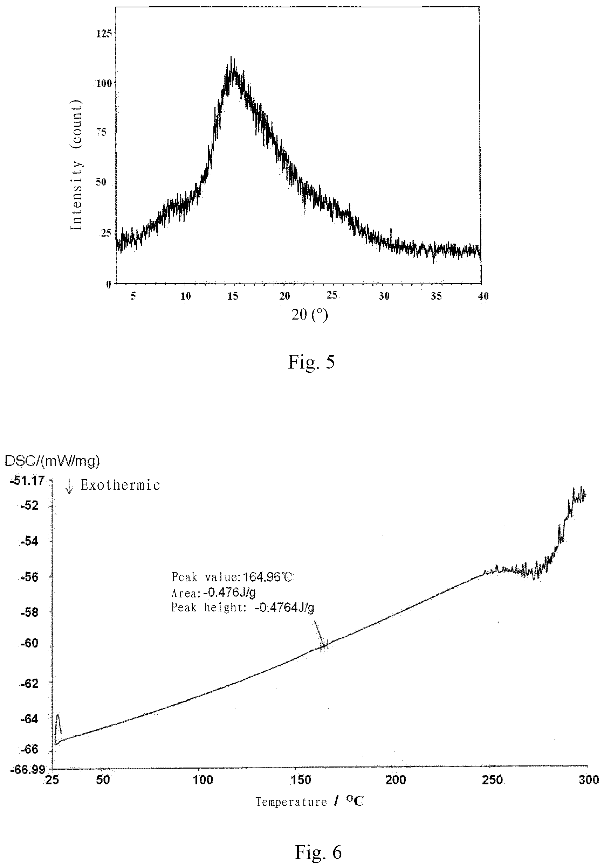

Generally, the mixture of A3/A4 oxime exists in an amorphous form, the typical X-ray powder diffraction pattern thereof is shown in Figure 5, and DSC absorption thermogram thereof is shown in Figure 6.

During the preparation of Milbemycin A3 oxime, the inventors of the present invention found that Milbemycin A3 oxime can form stable crystals. Comparing the DSC thermogram of the crystal form A of A3 oxime (Fig. 3) with the DSC thermogram of A3/A4 oxime (Fig. 6), the thermal stability of A3/A4 oxime was found to be much lower than that of the crystal form A of A3 oxime.

Meanwhile, the degradation stabilities of A3/A4 oxime and the crystal form A of A3 oxime were compared in the invention, and the degradation conditions are as follows:

### 1) Oxidative degradation

A sample of 50mg was weighed accurately and added to a 100.0ml volumetric flask, dissolved by adding 10ml methanol, 1.0 ml of 30% hydrogen peroxide was added, standing for 24 hours, then was diluted to the scale with methanol.

### 2) Photodegradation

According to the ICH requirement, the total illuminance requirement of 200 w·hr/m² and 1,200,000 lux·hr is required for photodegradation. If the illuminance is 600w/m², the ICH requirement is met, and the illumination time needs at least 9 hrs.

Approximately 600 mg of the sample was weighed, placed evenly in a weighing bottle, placed into a light box with an illuminance of 600 w/m² and illuminated for 24 hrs, taken out, placed into a desiccator and cooled to room temperature, and the weight loss on drying was measured.

### 3) Protection from light

About 600mg of the sample was weighed, placed evenly in a weighing bottle, the weighing bottle containing the sample was put into a black PE bag, and then the PE bag was packaged with an aluminum foil bag, the aluminum foil bag was placed into a light box with an illuminance of 600w/m². After illumination of 18 hrs, taken out, placed into a desiccator and cooled to room temperature, and the weight loss on drying was measured.

The HPLC conditions for content determination are as follows:

| | | | | |
|---|---|---|---|---|
| Column temperature: | room temperature (20-25°C) | | | |
| Injector temperature: | room temperature | | | |
| Detection wavelength: | 240nm | | | |
| Injection volume: | 20µl | | | |
| Flow rate: | 1.5ml/min | | | |
| Running time: | 50min | | | |
| Mobile phase A: | acetonitrile, which can be placed at room temperature for 1 year | | | |
| Mobile phase B: | 0.05% aqueous solution of trifluoroacetic acid (volume ratio), which can be placed at room temperature for 3 months | | | |
| | Detailed method of formulation: adding 1L of water to a 2L volumetric flask, adding 1.0ml of trifluoroacetic acid, adding water to the scale, covering and shaking evenly. | | | |

| Gradient elution | Time | Mobile phase A (%, volume percent) | Mobile phase B (%, volume percent) | Description |
|---|---|---|---|---|
| | 0-7 mins | 60 | 40 | Isocratic elution for 7min |
| | 7-42 mins | 60 - 75 | 40 -25 | Linear elution |
| | 42 - 45 mins | 75 - 60 | 25 - 40 | Rebalance |
| | 45-50 mins | 60 | 40 | Rebalance |

The results of degradation stability of A3/A4 oxime and the crystal form A of A3 oxime are shown in Table 2.

**Table 2**

| (%) | Content (A3/A4 oxime) | Total Impurity | Total Impurity increment | Contents (crystal form A of A3 oxime) | Total Impurity | Total Impurity increment |
|---|---|---|---|---|---|---|
| Original sample | 96.6 | 1.04 | / | 98.1 | 0.3215 | / |
| Oxidation | 95.8 | 1.49 | 0.45 | 98.3 | 0.2368 | -0.0847 |
| Illumination | 95.1 | 2.59 | 1.55 | 96.8 | 1.57 | 1.2485 |
| Protection from light | 96.37 | 1.04 | 0 | 96.9 | 0.3371 | 0.0156 |

The crystal form A of A3 oxime is superior to A3/A4 oxime in purity, and the above stability test results show that the stability of the crystal form A of A3 oxime is significantly better than that of A3/A4 oxime, so the crystal form A of A3 oxime is more convenient for storage.

## Claims

1. A crystal form A of Milbemycin A3 oxime, **characterized in that**, using Cu-Kα radiation, the crystal form A of Milbemycin A3 oxime has characteristic peaks at the following 2θ angles in the X-ray powder diffraction pattern: 5.32±0.20°, 8.39±0.20°, 13.98±0.20°, 14.45±0.20°, 15.36±0.20°, 16.50±0.20°, 18.61±0.20°, 25.95±0.20° and 26.28±0.20°.

2. The crystal form A of Milbemycin A3 oxime according to claim 1, wherein using Cu-Kα radiation, the crystal form A of Milbemycin A3 oxime further has characteristic peaks at the following 2θ angles in the X-ray powder diffraction pattern: 6.99±0.20°, 10.67±0.20°, 12.99±0.20°, 13.22±0.20°, 14.76±0.20°, 17.97±0.20°, 19.09±0.20°, 20.01±0.20°, 20.65±0.20°, 22.36±0.20°, 22.79±0.20°, 24.10 ± 0.20°, 27.42 ± 0.20° and 28.35 ± 0.20°.

3. A method of preparing the crystal form A of Milbemycin A3 oxime according to claim 1 or 2, **characterized in that**, the method includes the following steps:
(1) dissolving Milbemycin A3 oxime in a solvent to form a solution;
(2) adding the solution obtained in step (1) to an anti-solvent;
(3) crystallizing, filtrating and vacuum drying to obtain the crystal form A of Milbemycin A3 oxime.

4. The method according to claim 3, wherein the solvent is selected from methanol, ethanol, acetone, acetonitrile, DMF, n-propanol, isopropanol, n-butanol, isobutanol or chloroform, the anti-solvent is water or n-heptane.

5. The method according to claim 3 or 4, wherein the concentration of the Milbemycin A3 oxime in the solvent is 100 g/L to 500 g/L, preferably 200 g/L to 400 g/L.

6. The method according to any one of claims 3-5, wherein the temperature of the solution obtained in step (1) is controlled to be 50 °C to 80 °C, preferably 60 °C to 70 °C.

7. The method according to any one of claims 3-6, wherein after the solution obtained in step (1) is added to the anti-solvent, the volume content of the anti-solvent in the mixed liquid is 70 to 90%, preferably 75 to 85%.

8. The method according to any one of claims 3-7, wherein the temperature of the mixed liquid obtained in step (2) is controlled to be 30 °C to 60 °C, preferably 40 °C to 50 °C.

9. The method according to any one of claims 3-8, wherein prior to step (3), further including a step of adding seed crystals of the crystal form A of Milbemycin A3 oxime to the mixed liquid obtained in step (2).

10. The method according to any one of claims 3-9, wherein the vacuum drying temperature in step (3) is controlled to be 50 °C to 80 °C, preferably 60 °C to 70 °C, and the drying time is controlled to be 24-60h, preferably 36-48h.

11. Use of the crystal form A of Milbemycin A3 oxime according to any one of claims 1-10 in the preparation of an insecticide or acaricide.
